## Europäisches Patentamt

## European Patent Office

## Office européen des brevets

(11) Publication number: **0 047 097**
**B1**

(12) # EUROPEAN PATENT SPECIFICATION

(45) Date of publication of patent specification: **14.05.86**

(51) Int. Cl.⁴: **A 61 K 6/08**

(21) Application number: **81303759.5**

(22) Date of filing: **18.08.81**

(54) Visible light curable dental compositions.

(30) Priority: **29.08.80 US 182626**

(43) Date of publication of application:
**10.03.82 Bulletin 82/10**

(45) Publication of the grant of the patent:
**14.05.86 Bulletin 86/20**

(84) Designated Contracting States:
**AT BE CH DE FR GB IT LI NL SE**

(56) References cited:
**EP-A-0 012 535**
**FR-A-2 206 930**
**FR-A-2 370 467**
**US-A-4 097 994**

**KIRK-OTHMER: "Encyclopedia of chemical technology", Third Edition, vol. 1, 1978, pages 956-958, Wiley-Interscience Publication, New York (USA);**

(73) Proprietor: **DENTSPLY INTERNATIONAL INC.**
**570 W. College Avenue P.O. Box 872**
**York Pennsylvania 17405 (US)**

(72) Inventor: **Koblitz, Francis F.**
**31 N. Manheim Street**
**York Pennsylvania 17402 (US)**
Inventor: **Smith, Roy L.**
**505 Caulk Road**
**Milford Delaware 19963 (US)**

(74) Representative: **Newens, Leonard Eric et al**
**F.J. CLEVELAND & CO. 40/43 Chancery Lane**
**London WC2A 1JQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Background of the Invention

This invention provides composite dental restorative compositions which are curable by the action of visible light. More particularly, formulations are disclosed which maintain good colour stability and other beneficial physical properties while having the ease of workability and safety attendant to light curable materials.

Dental restoratives should exhibit certain obligatory physical and chemical characteristics in order to be suitable for use in filling, repairing or replacing teeth. These restorative materials should possess properties that closely match natural teeth with respect to structural properties such as cohesive strength index, coefficient of thermal expansion and wearability. Also aesthetic considerations such as colour stability, refractive index, plaque repellancy, polishability and opacity are important factors in determining whether a material is suitable for use as a dental restorative. In the past, numerous organic compositions have been tried in various mixtures and proportions in order to find satisfactory materials for use in dental composites or restoratives. These compositions have usually included some type of resin, which may be preblended or mixed by the practitioner in the office, together with other materials such as pigments, catalysts, handling agents and opacifiers. For restorative use, it is generally necessary to employ materials which are "filled", that is, to which have been added amounts of inorganic, or in some cases, organic particulate material.

Composite or restorative materials should be distinguished from most film forming dental compositions. Such film forming materials provide resins containing minor or no substantial amounts of filler materials. They are frequently used as sealants, glazes, bonding agents, or adhesives and may be used to coat a prepared tooth cavity prior to filling, thereby sealing off the tooth material against cracks and leaks adjacent to the filling. Such unfilled compositions have different viscosity requirements from dental composite materials because low viscosities are needed in the sealant materials in order to have proper flow characteristics. By contrast, dental composite and restorative materials must have good forming characteristics so that they can be shaped to fit a cavity area or molded into place in order to repair chipped or damaged teeth. Furthermore, such restorative compositions must preferably be filled with inorganic materials in order to achieve satisfactory hardness and durability.

It will be appreciated by those skilled in the art that the use of photoactivated materials is to be preferred over the more traditional thermochemical catalyst or redox activated systems because of the increased work time allowed by the use of photoinitiated polymerization. In a catalyst or redox system, work time is determined by the reaction time once the catalyst is added to the resin component. In a photocured system, the practioner may take whatever time is necessary for forming or molding the dental restoration into position and then effect extremely rapid curing by exposing the photocurable material to the appropriate wavelength of electromagnetic radiation.

Some prior art dental materials have utilized photoinitiators that are sensitive to ultraviolet light radiation. There are, however, certain technical limitations which are present in ultraviolet-activated dental composite or restorative systems. For example, tooth structure attenuates ultraviolet radiation sufficiently so that it is not practical to cure ultraviolet-activated dental composites where direct access to the dental composite by the ultraviolet source is interfered with by intervening portions of tooth structure. Such is the case in classical undercuts used for mechanical retention of dental restorations. Ultraviolet-cured systems also cannot accomplish good depths of cure; stepwise restoration is frequently required.

It has been found that visible light having wavelengths from about 4000 angstroms to about 5000 angstroms is attenuated to a lesser degree by tooth structure than is ultraviolet radiation. Accordingly, it has been proposed to employ such visible light as the source of activating energy in dental compositions. Many previous attempts to develop restorative formulations using visible light curing systems have resulted in failure; such previous compositions have failed to exhibit one or more of the servicability characteristics necessary for dental restoratives. A principal shortcoming is a lack of colour stability and concomitant lack of aesthetic acceptability of the resulting products. Such a·lack of colour stability is a major·shortcoming. The American Dental Association, the International Standards Organization, and others have developed detailed requirements for colour stability in direct filling resins. See, for example, A.D.A. Specification No. 27; J.A.D.A. vol. 94, June 1977, pp. 1191—1194.

Accordingly, it is a principal object of this invention to provide compositions which are useful as dental restoratives. It is another object to provide such dental materials which exhibit improved colour stability. Another object of this invention is to provide a one-component photocurable dental restorative system which is photocurable using visible light. A further object is to provide dental compositions which exhibit a rapid cure time but which exhibit good workability prior to curing. Yet another object is to provide photocurable dental restoratives which are curable without use of ultraviolet radiation. A still further object is to provide dental compositions which are effective with lesser amounts of photoinitiators. Still other objects will become apparent from the following description of the invention.

### Description of the Prior Art

It is to be understood that the term "bisphenol A" is commonly used in the art to indicate the chemical compound, 2,2-bis(4-hydroxyphenyl) propane. It is also to be understood that the term "bis-GMA" is commonly used to indicate the chemical compound 2,2-bis [4-(2-hydroxy-3-methacryloxypropoxy) phenyl] propane, otherwise referred to as "diglycidyl methacrylate of bisphenol A".

U.S. Patent No. 3,825,518 to Foster et al teaches dental filling materials which comprise a mixture of inorganic filler and the reaction product of an organic diisocyanate with a hydroxyalkyl acrylate or methacrylate to give a filled urethane diacrylate. A peroxide catalyst and a polymerization activator are employed.

U.S. Patent No. 3,254,411 to Shelley teaches the use of a polyurethane liner to form a coating in the area of tooth to be filled. This liner acts as a sealant to prevent seepage of food particles and bacteria behind the filling that is to be placed in the tooth.

U.S. Patent No. 3,488,269 to Allen et al teaches the use of visible light induced dye-redox initiated polymerization of vinyl monomers such as methacrylates. The improved initiators used here are characterized as having labile hydrogen.

U.S. Patent No. 3,864,133 to Disamatsu et al discloses photopolymerizable compositions which comprise a compound containing an ethylenically unsaturated double bond and urethane linkage and containing a secondary amino radical, a tertiary amino radical or a urea linkage. These materials are hardenable in the optional presence of air without the addition of wax. The materials formed are used in coating applications such as in wood sealers.

U.S. Patent No. 3,759,809 to Carlick et al describes radiation curable compositions that have at least one isocyanate-modified polyfunctional ester with a hydroxyl value of about 15 to 70 used in conjunction with a photoinitiator. Reduced toxicity of these compounds is also noted. The primary use of these compounds is in lithographic inks.

U.S. Patent No. 3,954,584 to Miyata et al teaches that certain photopolymerizable vinylurethane monomers are useful in the preparation of printing plates and reliefs. Actinic light having wavelengths of from 2000 to 5000 angstroms may be used depending on the species of the photosensitizers used. Photopolymerizable vinylurethane monomers are used in conjunction with a photo copolymerizable ethylenically unsaturated liquid monomer, and a photosensitizer, with the vinylurethane monomer.

U.S. Patent No. 4,065,587 to Ting utilizes UV curable poly(ether-urethane) polyacrylates for the formation of wet-lock polymers. U.S. Patent 3,968,181 to Uzelmeier teaches modified photocurable acrylate resins including bis-GMA. A UV sensitizer such as a benzoin ether may be employed. These materials are useful as coating and adhesives.

U.S. Patent 4,153,776 to Friedlander et al discloses amide-modified urethane acrylate radiation curable compounds which are useful in film applications.

U.S. Patent 4,071,424 to Dart et al teaches a photopolymerizable composition comprising at least one ethylenically unsaturated material and a photosensitive catalyst. Preferred photosensitizing systems employ alpha diketones with an amine reducing agent capable of being excited by radiation in the visible as well as in the UV region or both.

U.S. Patent 4,089,762 to Frodsham teaches the use of a photopolymerizable composition comprising a polymerizable ethylenically unsaturated material, a photosensitizer of the structure which is preferably a diketone and a N-alkyl or N-cycloalkyl morpholine.

U.S. Patent 4,089,763 to Dart et al teaches a method of repairing teeth using a composition which is curable by irradiation with visible light. These compositions include isocyanate modified bisphenol A derivatives in conjunction with a visible light sensitizing system.

U.S. Patent 3,709,866 to Waller, assigned to the assignee of this invention, discloses the hexamethylene diisocyanate adduct of bis-GMA in a UV photocurable system. Benzoin methyl ether is employed as a UV activator. This disclosure is in part reflected in the commercial products NUVA-FIL, NUVA-FIL P.A., NUVA-SEAL, NUVA-SEAL P.A. which are registered trademarks for products of the L. D. Caulk Co.

Published French Patent Application No. 2 370 467 discloses visible light curable dental restorative compositions which all employ unmodified bis-GMA together with a diluent acrylic monomer, a photo initiator and a photo accelerator. The exemplified initiators include the aromatic alpha diketones benzil, 4, 4'-oxydibenzil and 4,4'-dimethoxybenzil while the exemplified photoaccelerators are all tri-alkyl amines.

It has been discovered that visible light polymerizable dental restorative compositions may be formulated having the required physical characteristics for filling teeth together with improved colour stability. Such

a moldable dental restorative composition comprising a blend of:

a major proportion of an inorganic filler, and

a minor proportion of a resin component comprising:

a binder resin,

a polymerizable acrylic diluent monomer present in an amount sufficient to dissolve said binder resin, and

a photosensitizing system comprising:

a non-aromatic alpha-diketone, and an amine reducing agent in an amount sufficient to improve the photocatalytic activity to visible light of said alpha-diketone in said restorative composition, is characterised in that the binder resin comprises the reaction product of bis-GMA and a diisocyanate and the alpha-diketone comprises from about 0.05% to about 0.5% by weight of said resin component.

Such compositions may optionally be modified through the addition of restorative modificants such as pigments, stabilizers, opacifiers and handling agents as will be appreciated by those skilled in the art. These materials are freely workable under ambient conditions until they are exposed to visible light. At such time a rapid cure is effected to yield strong, durable, polishable dental restoratives with improved colour stability.

In embodiments of the invention, bis-GMA may be reacted with a diisocyanate such as hexamethylene diisocyanate, phenylene diisocyanate or a wide variety of other aliphatic and aromatic diisocyanates to provide useful binder resins. The adducts of bis-GMA with hexamethylene diisocyanate has been found to be the best binder resin presently known for use in this invention.

The acrylic diluent monomer is added to the compositions of this invention in amounts sufficient to result in polymerizable formulations usually having viscosities between about 5,000 and about 70,000 mPas (centipoises), and preferably between about 40,000 and about 60,000 mPas (centipoises). Other viscosities may be employed for certain embodiments, however, such viscosity control will be understood by those skilled in the art to result in moldable, workable, thixotropic materials suitable for a wide range of dental restorative uses.

Preferably the diluents monomer may be the di-, tri- or higher acrylic species such as ethylene glycol dimethacrylate, diethylene glycol dimethacrylate, triethylene glycol dimethacrylate, and trimethylol propane trimethacrylate.

While the components of the polymerized blend may be added in any order, it has been found useful and convenient to mix the binder resin and diluent together, to add the photosensitizing system components, and then to blend in the filler together with pigments and other modifying agents. In practice, the binder resin and diluent are mixed together in a proportion such that the final polymerizable composition will have the desired viscosity. While the relative amounts of binder resin and diluent will vary depending upon their identity and the type, size and amount of filler to be used, ratios of binder resin to diluent of from about 12:1 to about 1:2 and more preferably, of from 6:1 to about 1:1 are generally employed.

While any non-aromatic alpha-diketone which is capable of initiating polymerization in the polymerizable systems of this invention may be employed, camphoroquinone and biacetyl, have been found to be preferred. Most preferred is camphoroquinone.

The non-aromatic alpha-diketone is combined with an amine reducing agent; the two taken together form the visible light sensitizing system for the practice of this invention. Numerous amines have been found to be useful as reducing agents for the alpha diketones used herein. Thus, amines such as tributylamine and tripropylamine are useful. Still more useful are substituted amines such as N-alkyl dialkanolamines and trialkanolamines, N-methyl diethanolamine is most preferred.

The improved colour stability of the dental restoratives according to this invention is believed to be at least in part a result of the careful control of the amounts of non-aromatic alpha-diketone sensitizers employed therein. It is even more preferred to employ from about 0.10% to about 0.30% of alpha-diketone. The amount of amine reducing agent is less critical. It is useful to employ from about 0.2% to about 1.0% of amine with from about 0.3% to about 0.6% being preferred based on the total weight of binder, diluent and photosensitizing system.

For the formulation of the visible light polymerizable compositions of this invention, the binder resin, diluent and visible light photosenstizing system are blended together with fillers and, optionally but preferably, pigments and modificants. Those skilled in the art will appreciate that the amount of filler loading which may be accomplished with a given resinous system will depend upon several variables including the identity of the resins and fillers and the particle sizes of the fillers. The filler used must be such that the transmittance of visible light by the restorative compositions is sufficient for polymerization to take place. Those skilled in the art will be able to select fillers and to determine filler particle sizes based upon this requirement.

Among those fillers which are especially suited for use in the practice of this invention are inorganic glasses. Preferred among these are barium aluminium silicate, lithium aluminium silicate, strontium, lanthanum and tantalum, glasses. Silica, especially in submicron sizes, quartz, and other fillers may also be employed in some formulations. Such fillers are preferably silanated prior to use in the restoratives of this invention. Silanation is well known to those skilled in the art and any silanating compound known to them may be used for this purpose.

Fillers are selected having particle sizes which are, in general, less than about 50 microns. It is known that smaller sized filler particles result in highly polishable dental materials, but that the concomitant increase in surface area diminishes the overall filler loading possible with a given resin. Such lower loadings may be manifested by lesser degrees of strength, hardness, and durability in the resulting polymerized structures. It is possible to employ submicron sized fillers in some cases, however, the ratio of resin to filler employed for the practice of this invention must take account of the filler size as suggested above. Weight ratios of from 15:85 to 30:70 are preferred.

The methods of use of the visible light curable compositions of this invention follow, to an extent, those currently practiced by those skilled in the art. Thus, the surface to be repaired is cleansed of decayed material and acid etched to promote bonding. At this point, a bonding agent may be employed by coating it upon the surface to be repaired. A material according to this invention is then molded into place in the conventional fashion. At this point, visible light is directed onto the restorative material by any suitable source.

One such source is described in the application of Gonser "Visible Light Apparatus for Curing Photo-Curable Compositions", assigned to the Assignee of this invention and accorded Serial No. 182,643, filed 29th August, 1980 (EP—A—46939).

This exposure may take place directly or through one or more surfaces of tooth material due to the significant transmittance of tooth material to visible light. Following exposure, the restorative material undergoes polymerization. During this process, and afterward, the materials of this invention exhibit colour stability in accordance with A.D.A. Specification 27.

The following non-limiting examples further illustrate certain preferred embodiments of this invention.

Example 1
Visible Light Curable Composition

A resinous blend was formed from 13.57 g of the hexamethylene diisocyanate adduct of bis-GMA, 6.79 g of triethylene glycol dimethacrylate, 0.03 g of camphoroquinone and 0.1 g of N-methyl diethanolamine. To this resinous component blend were added fillers and pigments comprising 78.02 g Raysorb® T3000, a radiopaque silanated barium glass filler offered by the Kimble Company, 0.6 g of silanated alumina, 0.002 g yellow iron oxide, 0.005 g red iron oxide, 0.134 g of a dispersion of carbon black or barium glass and 0.75 g of Tullanox® 300 which is a submicron silica available from the Tulco Co. The resulting filled blend is useful as a composite or restorative and is curable by exposure to visible light. This dental composition is suitable for a wide range of dental restorative uses and passes A.D.A. Specification Number 27 for colour stability.

Example 2
Polymerized Dental Restorative

The composition of Example 1 was exposed to visible radiation having wavelengths generally between about $4 \times 10^{-7}$ m and about $5 \times 10^{-7}$ m at an intensity of about 430 mw/cm². Exposure times of about 10 seconds resulted in depths of cure of about 2.5 mm. Longer exposures effected deeper and more complete cures. Exposures of about 30 seconds are sufficient for most restorative uses and exposure either through the composite or through tooth material is effective in consumating polymerization. Teeth may be cleaned, etched and filled in the conventional way with the composition of Example 1. Irradiation of such preparations in various restorative configurations yields well-formed, colour stable, strong, and durable restored structures.

Claims

1. A moldable dental restorative composition hardenable by irradiation with visible light comprising a blend of:

a major proportion of an inorganic filler, and
a minor proportion of a resin component comprising:
a binder resin,
a polymerizable acrylic diluent monomer present in an amount sufficient to dissolve said binder resin, and
a photosensitizing system comprising:
a non-aromatic alpha-diketone, and an amine reducing agent in an amount sufficient to improve the photocatalytic activity to visible light of said alpha-diketone in said restorative composition, said inorganic filler and said resin component being present in relative amounts such that said restorative composition has a viscosity suitable for filling teeth; said composition being characterised in that the binder resin comprises the reaction product of bis-GMA and a diisocyanate and the alpha-diketone comprises from 0.05% to 0.5% by weight of said resin component.

2. A composition is claimed in Claim 1 characterised in that the alpha-diketone is biacetyl.

3. A composition as claimed in Claim 1 characterised in that the alpha-diketone is camphoroquinone.

4. A composition as claimed in Claim 1 characterised in that the amine is either a N-alkyldialkanolamine or a trialkanolamine.

5. A composition as claimed in Claim 1 characterised in that the organic filler is selected from inorganic glasses and silica.

Revendications

1. Composition de restauration dentaire moulable, durcissable par irradiation avec de la lumière visible, comprenant un mélange:
d'une proportion importante d'une charge inorganique et
d'une petite proportion d'un composant résineux comprenant:
une résine de liaison,
un monomère acrylique polymérisable comme diluant présent en une quantité suffisante pour dissoudre la résine de liaison, et
un système de photosensibilisation comprenant:
une alpha-dicétone non aromatique et un agent de réduction aminé en une quantité suffisante pour améliorer l'activité photocatalytique à la lumière visible de l'alpha-dicétone dans la composition de restauration précitée, la charge inorganique et le composant résineux précités étant présents en des quantités relatives telles que la composition de restauration présente une viscosité appropriée à l'obturation des dents,
cette composition étant caractérisée en ce que la résine de liaison comprend le produit de réaction de bis-GMA et d'un diisocyanate et en ce que l'alpha-dicétone constitue de 0,05% à 0,5% en poids du composant résineux précité.

2. Composition suivant la revendication 1, caractérisée en ce que l'alpha-dicétone est le biacétyle.

3. Composition suivant la revendication 1, caractérisée en ce que l'alpha-dicétone est la camphoroquinone.

4. Composition suivant la revendication 1, caractérisée en ce que l'amine est soit une N-alkyldialcanolamine soit une trialcanolamine.

5. Composition suivant la revendication 1, caractérisée en ce que la charge inorganique est choisie parmi les verres organiques et la silice.

**Patentansprüche**

1. Gießbares zahnärztliches Restaurationspräparat, das durch Bestrahlung mit sichtbarem Licht aushärtbar ist, bestehend aus einer Mischung aus:

einem größeren Gehalt an anorganischem Füllmaterial, und

einem kleineren Gehalt an einer Harzkomponente, bestehend aus:

einem Binderharz,

einem polymerisierbaren acrylischen Verdünnungsmonomer, das in einem Anteil vorhanden ist, der ausreichend ist, um das Binderharz zu lösen, und

einem photosensibilisierenden System, enthaltend:

ein nichtaromatisches Alphadiketon und ein Amin-Reduzierungsmittel in einem Anteil, der ausreichend ist, um die photokatalytische Aktivität des Alphadiketons in dem Restaurationspräparat für sichtbares Licht zu verbessern, wobei das anorganische Füllmaterial und die Harzkomponente in solchen relativen Anteilen vorhanden sind, daß das Restaurationspräparat eine zum Füllen von Zähnen geeignete Viskosität hat; dadurch gekennzeichnet, daß in dem Präparat das Binderharz das Reaktionsprodukt von Bis-GMA und einem Diisocyanat enthält und das Alphadiketon von 0,05 bis 0,5 Gew.-% der Harzkomponente ausmacht.

2. Ein Präparat nach Anspruch 1, dadurch gekennzeichnet, daß das Alphadiketon Biacetyl ist.

3. Ein Präparat nach Anspruch 1, dadurch gekennzeichnet, daß das Alphadiketon Kampferchinon ist.

4. Ein Präparat nach Anspruch 1, dadurch gekennzeichnet, daß das Amin entweder ein N-Alkyldialkanolamin oder ein Trialkanolamin ist.

5. Ein Präparat nach Anspruch 1, dadurch gekennzeichnet, daß das anorganische Füllmaterial aus anorganischen Gläsern und Aluminiumhydroxyd ausgewählt ist.